# EUROPÄISCHE PATENTANMELDUNG

(11) **EP 1 186 273 A2**
(43) Veröffentlichungstag der Anmeldung: **13.03.2002**
(21) Anmeldenummer: 01121348.5
(22) Anmeldetag: 06.09.2001
(51) Int. Cl.: A61B 5/16

(54) **Vorrichtung zur Vitalitätsdiagnostik**

(30) Priorität: 06.09.2000 DE 20015449 U
(71) Anmelder: Gruber, Richard, Dr. med., 86825 Bad Wörishofen (DE)
(72) Erfinder: Gruber, Richard, Dr. Med., 86825 Bad Wörishofen (DE)
(74) Vertreter: Hiebsch, Gerhard F., Dipl.-Ing.

(57) **Zusammenfassung**

Die Erfindung betrifft eine Vorrichtung zur Vitalitätsdiagnostik an einer Testperson, mit einer einer Datenverarbeitungsvorrichtung (12) zugeordneten Ausgabeeinheit (14, 18), die zum Ausgeben eines visuellen und/oder akustischen Signals für die Testperson ausgebildet ist, sowie einer Betätigungs- und Sensoreinrichtung (16, 18), die zum Erfassen einer Eingabebetätigung der Testperson als Reaktion auf das visuelle oder akustische Signal eingerichtet ist. Ein Ausgangssignal der Betätigungs- und Sensoreinrichtung wird durch die Datenverarbeitungsvorrichtung datenmäßig erfasst und zur Ermittlung vitalitätsrelevanter Daten weiter verarbeitet, wobei die Betätigungs- und Sensoreinrichtung sowie die Ausgabeeinheit einen gemeinsamen Berührungs- und/oder druckempfindlichen Bildschirm (18) aufweisen und die Datenverarbeitungsvorrichtung mit dem Bildschirm in einem Gehäuse (10) integriert ist.

## Beschreibung

Die vorliegende Erfindung betrifft eine Vorrichtung zur Vitalitätsdiagnostik, wie sie etwa in Form von zum Anschluss an einen handelsüblichen PC vorgesehenen Peripheriegeräten bekannt ist, die, in einem geeigneten Gehäuse, sowohl eine (zumeist optische) Ausgabe- bzw. Anzeigeeinheit in Form einer oder mehrerer Leuchtelemente aufweist, und diesen Leuchtelementen dann Betätigungselemente, zumeist Tasten od. dgl. zugeordnet sind, so dass durch geeignete Softwarekonfigurierung des PC dann insbesondere Reaktionszeiten eines mit einem solchen Gerät arbeitenden Probanden gemessen werden können, etwa der Zeitablauf zwischen dem Erleuchten eines Leuchtsignals und dem Betätigen des zugehörigen Tasters durch die Testperson.

Durch geeignete Auswertung dieser Daten und insbesondere durch Vergleich mit anderen Testpersonen können so Feststellungen über die Vitalität des Probanden getroffen werden, wobei der Begriff "Vitalität" (oder "functional age" bzw. "altersabhängige Vitalfunktion") als Funktions-Analyse altersabhängiger Organfunktionen im Rahmen der vorliegenden Anmeldung weit auszulegen ist und prinzipiell beliebige altersrelevante bzw. physische Parameter umfasst, die sich als Reaktion der Testperson auf einen akustischen, visuellen, sensuellen o. ä. Reiz messen lassen.

Geeignet aufbereitet, und insbesondere nach Alter, Geschlecht od. dgl. Maßstäben klassifiziert, lassen sich somit aus den mit gattungsgemäßen Vorrichtungen messbaren Reaktionen wertvolle Rückschlüsse gewinnen, die insbesondere einer Testperson eine Orientierung bzw. Einordnung seiner/ihrer Leistungsfähigkeit in eine Bezugsgruppe ermöglicht.

Genauer gesagt basieren derartige Maßnahmen zur Vitalitätsdiagnostik auf einer Mehrzahl von Einzeltests, die möglichst integrative Altersveränderungen erfassen, d.h. auf der hierarchischen Ebene von Funktionssystemen liegen (auch daher der Begriff des "functional age"). Entsprechend ist es daher durch geeignete Auswahl der Tests möglich, nicht nur, entsprechend den komplexen Anforderungen, die oft asynchronen Alternsveränderungen des physischen, mentalen, emotionalen und sozialen Bereiches möglichst ganzheitlich in der genannten Vielfältigkeit zu berücksichtigen, auch bietet eine Realisierung in Form einer kombinierten Hard- und Softwareumgebung die beste Voraussetzung für Objektivität, Reliabilität und Validität des Gesamtverfahrens der durchgeführten Untersuchungen. Nicht zuletzt erweist es sich bei gattungsgemäßen Ansätzen als vorteilhaft, dass es sich um nichtinvasive Maßnahmen handelt, die sich zudem für breiten ambulanten Einsatz in Arztpraxen, Kliniken, Instituten, Kurzentren, aber auch zur alternativen Erschließung von ärztlichen präventiven Angeboten eignen.

Handelsübliche Vitalitätsdiagnostikvorrichtungen basieren dabei primär auf relativ einfachen Reaktionszeitmessungen, wobei der jeweils die Reaktionszeit auslösende Reiz akustischer Natur sein kann (also etwa über einen Lautsprecher oder Kopfhörer der Testperson angebotenes Akustiksignal), ergänzend und/oder alternativ ein visuelles Signal (etwa ein Aufleuchten einer Leuchtdiode), oder ein mit dem Tastsinn fühlbares Signal, wie es etwa durch eine (z. B. elektrisch betriebene) Vibrationseinheit erzeugt wird. Gemessen werden können dann insbesondere Zeiten zwischen der Erzeugung des Reizsignals und einer Reaktion der Testperson, wobei diese Messung entweder eine reine Reaktionszeit ist (wobei dann maßgeblich ist, innerhalb welcher Zeit die Testperson auf das Reizsignal reagiert), oder aber das Reizsignal selbst variiert wird (und dann gemessen werden kann, ab welcher Lautstärke und/oder Frequenz etwa die Testperson einen Ton wahrnimmt, ab welcher Vibrationsamplitude eine Vibration gefühlt wird usw.).

Im Ergebnis ermöglichen daher handelsübliche Vorrichtungen, die gängigerweise mit geringem hardwaretechnischem Aufwand realisierbar sind, bereits das Abdecken einer recht großen Anzahl von vitalitätsrelevanten, zu einer Diagnose geeigneten Tests.

Angesichts des in jüngerer Zeit spürbar zunehmenden Interesses an derartigen Vorrichtungen stellt sich jedoch heraus, dass die bekannten, als simple Peripheriegeräte zu handelsüblichen Desktop-PCs realisierten Vorrichtungen gerade einem Dauereinsatz in einer auf Fragen der Altersheilkunde spezialisierten Praxis kaum gewachsen sind. So bereiten bereits die zahlreichen, notwendigen Verkabelungen, etwa zwischen dem eigentlichen Diagnosegerät und dem PC sowie zwischen etwaigen, wiederum mit dem Diagnosegerät verbundenen Sensorikeinheiten, zahlreiche Installations- und Verbindungsprobleme, insbesondere für ungeübtes Personal, und auch im Dauerbetrieb besteht hier die Gefahr von Fehlverbindungen, Verschmutzungen od. dgl.. Hinzu kommen die gerade in einem ärztlichen Umfeld verschärften Hygieneanforderungen, denen oftmals in Form konventioneller Peripheriegeräte erstellte und verkabelte Vitalitätsdiagnostikvorrichtungen kaum gerecht werden.

Durch die Notwendigkeit, gattungsgemäße Peripheriegeräte mit prinzipiell beliebigen PCs zusammenarbeiten zu lassen, entstehen zudem häufig Kompatibilitätsprobleme, die wiederum den typischen, nicht datentechnisch geschulten Anwender überfordern.

Ferner stellt es sich im praktischen Betrieb als nachteilig heraus, dass die durch gattungsgemäße, extern an PCs anzuschließende Boxen realisierte Diagnostikgeräte angebotenen visuellen Darstellungsmöglichkeiten bei der Erzeugung von Testsignalen äußerst beschränkt sind, so dass es wünschenswert wäre, im Interesse einer Verbreiterung der Testmöglichkeiten hier auch eine größere Flexibilität bei der Erzeugung visueller (insbesondere auch akustischer) Signale zu erhalten; entsprechendes gilt für die Möglichkeiten eines Probanden, mit der Vorrichtung zu interagieren.

Entsprechend ist es Aufgabe der vorliegenden Erfindung, eine gattungsgemäße Vorrichtung zur Vitalitätsdiagnostik sowohl im Hinblick auf ihre praktischen Gebrauchseigenschaften in Installation, Betrieb, Wartung und Hygiene zu verbessern, als auch flexiblere, bedienungsfreundlichere Möglichkeiten bei der Erzeugung von Reizsignalen für einen Probanden sowie in der Erfassung einer Probandenreaktion darauf zu schaffen.

Die Aufgabe wird durch die Vorrichtung mit den Merkmalen des Anspruchs 1 gelöst; vorteilhafte Weiterbildungen der Erfindung sind in den Unteransprüchen beschrieben.

So ist es ein charakteristisches Merkmal der vorliegenden Erfindung, die Ausgabeeinheit (die traditionell z. B. aus einer oder mehreren LEDs bestand), durch einen (einem PC zugeordneten) Bildschirm zu realisieren und diesen Bildschirm zudem druckempfindlich auszugestalten, so dass durch diese Druck- bzw. Berührungsempfindlichkeit gleichzeitig die Aufgabe der Betätigungs- und Sensoreinrichtung, die traditionell mit Formen separater Taster od. dgl. gelöst werden muss, übernommen werden kann.

Die sich hieraus ergebenden Vorteile sind evident: Nicht nur gibt es nunmehr mit vergleichsweise einfachen softwaretechnischen Mitteln die Möglichkeit, visuelle Ausgabesignale in Form von geeignet geformten und auf dem Bildschirm plazierten Flächen in beliebiger Weise, Reihenfolge, Farbe usw. zu erzeugen, auch bietet der erfindungsgemäß eingesetzte, berührungsempfindliche Bildschirm die Möglichkeit, diese (oder andere) Teilflächen auf dem Bildschirm als Detektoren für eine Berührung (und damit eine Reaktion auf den Reiz) auszuwerten. Prinzipiell kann dabei die Darstellbarkeit bzw. Positionierung, wie gesagt, beliebig sein.

Wird dann noch, wie von der Erfindung vorgesehen, die Vitalitätsdiagnostikvorrichtung als Einheit in ein einziges Gehäuse integriert, so ergibt sich eine einfach anzuschließende, einfach zu reinigende bzw. zu desinfizierende und gegen Störungen (wie etwa abfallende Kabel od. dgl.) unempfindliche Anordnung, die sich insbesondere für einen Dauereinsatz in geeigneten Diagnostikbetrieben eignet: Im Idealfall interagiert eine Testperson lediglich noch mit dem ihr (üblicherweise in einem Pultgehäuse) angebotenen berührungsempfindlichen Flachbildschirm, so dass weder zusätzliche Bedienknöpfe oder -taster die Testperson verwirren, noch hier potentielle Probleme hinsichtlich Verschmutzung oder elektrischem Kontakt bestehen.

Dabei ist es im Rahmen der vorliegenden Erfindung jedoch nicht ausgeschlossen, dass die in einem Gehäuse integrierte Vorrichtung zusätzlich mit extern angebundenen, manuell handhabbaren Sensorik- bzw. Ausgabeeinheiten zusammenwirkt: So ist im Rahmen der vorliegenden Erfindung insbesondere eine Handkraftmessung vorgesehen, wofür eine manuell handhabbare, von dem Gehäuse der Vitalitätsdiagonstikvorrichtung lösbare Handeinheit mit einem geeignet konfigurierten Drucksensor bei einem Zusammenpressen der Handeinheit durch die Testperson die Maximalkraft erfasst und ein entsprechendes elektronisches Signal (bevorzugt drahtlos) zum Hauptgerät zur weiteren Auswertung und Darstellung zurückleitet. Entsprechendes gilt für zusätzlich anschließbare Ausgabeeinheiten, etwa eine (typischerweise geeignet mittels einer Schwingspule realisierten) handhabbaren Vibrationseinheit, welche dann den von der Testperson zu erfühlenden Vibrationsreiz anbietet.

Für die Anbindung derartiger, manuell handhabbarer Einheiten (die weiter bevorzugt jeweils ein leicht pflegbares, eloxiertes Aluminiumgehäuse oder Kunststoffgehäuse aufweisen) hat sich eine Infrarotanbindung, etwa mittels des IrDA-Standards, bewährt.

Da zudem durch die Verwendung eines berührungsempfindlichen Bildschirms als (visuelles) Ausgabemedium prinzipiell die Art, Farbe und Anzahl von zur Reaktion einer Testperson angebotenen optischen Reizflächen unbeschränkt ist, bietet die vorliegende Erfindung zahlreiche neue diagnostische Möglichkeiten, etwa das Versehen einer Mehrzahl von auf dem Bildschirm erzeugten visuellen Flächenelementen mit Reihenfolgekennern (z. B. Nummern), wobei dann mit der erfindungsgemäßen Vorrichtung erfasst werden kann, in welcher Zeit eine Testperson durch Berühren der jeweiligen Teilflächen in der durch die Kennung vorbestimmten Reihenfolge die korrekte Reihenfolge wieder herstellen kann.

Ein weiterer Vorteil des erfindungsgemäß eingesetzten berührungsempfindlichen Bildschirmes liegt darin, dass dieser grundsätzlich neue, komfortable Möglichkeiten zur Benutzerführung bzw. Erläuterung von jeweiligen Testmaßnahmen gegenüber der Testperson ermöglicht. So ist es im Rahmen einer bevorzugten Weiterbildung der Erfindung insbesondere vorgesehen, der Testperson (automatisch oder nach Betätigen einer entsprechenden Anforderungsfläche) geeignete Hilfe-oder Anleitungstexte anzubieten, oder gar animierte Darstellungen in Form einer weiter bevorzugt mit einem Audiosignal, etwa einem gesprochenen Erklärungstsext, unterlegten Videosequenz, die in illustrativer, die Akzeptanz von jeweils vorgenommenen Tests deutlich erhöhenden Weise den Benutzer über die von ihm erwarteten Reaktionen (bzw. etwaige Hintergründe) informiert; eine weitere positive Wirkung liegt darin, dass bei Nutzung dieser audio-visuellen Erläuterungsmöglichkeiten der notwendige Erklärungsaufwand durch eine Bedienperson drastisch minimiert, wenn nicht gar auf Null reduziert werden kann.

Besonders bevorzugt ist es zudem, die erfindungsgemäße Vorrichtung mit einer Datenkommunikationsschnittstelle zu versehen, die, typischerweise über das Internet oder ein internes Intranet, eine Kommunikation mit einem zentralen Daten- und Updateserver ermöglicht: So ist es damit nicht nur erreichbar, dass eine den bestimmungsgemäßen Betrieb der Datenverarbeitungsvorrichtung ermöglichende Software stets auf einem aktuellen Stand gehalten wird, insbesondere ermöglicht diese Anbindung an die Servereinheit auch den Austausch der jeweils gemessenen Probandendaten sowie eine Aktualisierung der jeweiligen Bezugsdaten, so dass sowohl dann der lokale Nutzer der Vitalitätsdiagnostikvorrichtung einen aktuellen, vollständigen Satz von Bezugsdaten besitzt, innerhalb derer dann ein aktueller Probandenwert eingeordnet werden kann, als auch die jeweils jüngst gemessenen, aktuellen Probandenwerte dann der zentralen Servereinheit zum Aktualisieren der Datengesamtheit zurückfließen.

Während bereits die Bildschirmeinheit eine illustrative und komfortable Rückmeldung der jeweils gemessenen Daten (einschließlich einer etwaigen graphischen Aufbereitung) ermöglicht, so dürfte es sich oft als wünschenswert herausstellen, einer Testperson ihre jeweils gemessenen Werte (ggf. einer optischen Aufbereitung bzw. Einordnung in die Bezugsgruppenwerte) in ausgedruckter Form anzubringen, so dass gemäß einer bevorzugten Weiterbildung der Erfindung eine zugehörige, bevorzugt Farbdruck ermöglichende Druckeinheit Bestandteil des Gehäuses ist, oder aber ein ansonsten bekannter Farbdrucker in einfacher Weise mit dem Gehäuse verbunden werden kann.

Weitere Vorteile, Merkmale und Einzelheiten der Erfindung ergeben sich aus der nachfolgenden Beschreibung bevorzugter Ausführungsbeispiele sowie anhand der Zeichnung; diese zeigen in:
- Fig. 1:: ein Blockschaltbild der erfindungsgemäßen Vorrichtung zur Vitalitätsdiagnostik gemäß einer ersten, bevorzugten Ausführungsform der vorliegenden Erfindung,
- Fig. 2:: eine exemplarische Darstellung verschiedener Reaktions- und Erläuterungstextflächen auf der berührungsempfindlichen Bildschirmeinheit gemäß Blockschaltbild in Fig. 1 und
- Fig. 3:: eine Explosionsansicht mit dem Gehäuse sowie den Handeinheiten in der Ausführungsform gemäß Fig. 1 in einer Explosionsdarstellung.

Ein im beschriebenen Ausführungsbeispiel aus lackiertem Aluminium oder Kunststoff gefertigtes, pultförmiges Gehäuse 10 legt auf seiner einem Benutzer zugewandten Schrägfläche einen im Gehäuse montierten druckempfindlichen Bildschirm 18 frei, der in ansonsten bekannter Weise von einer ebenfalls im Gehäuse 10 vorgesehenen PC-Systemeinheit angesteuert wird, wobei insbesondere durch geeignete Software-Programmierung der Systemeinheit 12 auf der Bildschirmeinheit 18 beliebige Ausgabe- und Reaktionssignale, eingeschlossen Bedienflächen, sowie Texte und/oder Bilder darstellbar sind.

Durch im beschriebenen Ausführungsbeispiel geeignete Softwareprogrammierung der Systemeinheit 12 sowie (ansonsten bekannte) Schnittstellenmodule sind im Gehäuse 10 zusätzlich eine Ausgabeeinheit 16 sowie eine Betätigungs- und Sensoreinheit 14 realisiert, die in nachfolgend zu beschreibender Weise sowohl die Bildschirmeinheit 18 als Ausgabe- bzw. Eingabemedium für Reizsignale und Reaktionen an einer Testperson darauf ansprechen, als auch spezielle Funktionen zu angeschlossenen externen Handeinheiten 20 bis 26 wahrnehmen.

Genauer gesagt ist die handbetätigbare Einheit 20 als Vibrationsgeber ausgebildet und weist eine in einem glockenförmigen Aluminiumgehäuse zum Umgreifen mit der Hand der Testperson vorgesehene Schwingspule auf, die durch geeignete Ansteuerung der Systemeinheit 12 bzw. der Ausgabeeinheit 16 zum Schwingen mit einer vorbestimmten, veränderbaren Frequenz sowie einer veränderbaren Amplitude ansteuerbar ist. Zweck dieser mittels einer Infrarot-Schnittstelle an die Ausgabeeinheit 16 angebundenen Vibrationseinheit 20 ist es, einer Testperson ein zu ertastendes Vibrationssignal anzubieten und den Zeitpunkt (und indirekt damit etwa Frequenz und/oder Amplitude) zu messen, bei welchem die Testperson auf das Vibrationssignal reagiert.

Einen analogen Gedanken verfolgt die handbetätigbare Einheit 22, die, ebenfalls über eine Infrarotverbindung (IR) drahtlos angebunden, das Sehvermögen einer Testperson überprüfen kann: Die Einheit 22 enthält eine hinter einem Okular im glockenförmigen Aluminiumgehäuse durch einen Stellmotor geeignet bewegbare Optik, welche relativ zu einer Ziffern-Leuchtanzeige (im beschriebenen Ausführungsbeispiel die leicht verwechselbaren und daher gut für eine Augendiagnose geeigneten Ziffern 0 und 8) bewegbar ist und somit eine Fokussierungswirkung für die durch das Okular die Leuchtziffern betrachtende Testperson erzeugt. Indem dann die Testperson durch Berühren einer vorbestimmten Detektorfläche auf dem Bildschirm 18 das positive Erkennen einer Ziffer anzeigt, lässt sich die Akkomodationsbreite des Auges des Probanden ermitteln, mithin also eine Sehschärfemessung vornehmen.

Die manuell handhabbare Einheit 24 ist zum Zweck der Handkraftmessung, wiederum mittels einer Infrarot-Verbindung, drahtlos mit der Betätigungs- und Sensoreinheit verbunden: In Gehäuseform und -gestaltung vergleichbar den Einheiten 20, 22, weist das Aluminiumgehäuse der Handkrafteinheit 24 einen Druckaufnehmer auf, der eine bevorzugt zwischen zwei Gehäusehälften der Einheit 24 wirkende maximale Kraft aufnimmt und so die Erzeugung eines Messwertes für die Handkraft der Testperson ermöglicht. Zu diesem Zweck bietet es sich an, das Aluminiumgehäuse durch Vorsehen eines oder mehrerer Schlitze geeignet vorzubereiten.

Schließlich ist die manuell handhabbare Einheit 26 als Lungenfunktionstesteinheit ausgebildet und ermöglicht einen Test der Lungenfunktion dadurch, dass die Testperson durch ein geeignet am -- wiederum glockenförmigen -- Gehäuse vorgesehenes Mundstück Atemluft einbläst, im Gehäuse ein Druckaufnehmer für den durch das Einblasen entstehenden Staudruck vorgesehen und durch Vergleich mit dem Umgebungsdruck eine Messung einer Atemdruckkurve über die Zeit möglich ist.

Wie auch bei den anderen manuell handhabbaren Einheiten 20 bis 24 erfolgt der Betrieb bzw. die Benutzung der Lungenfuktionstesteinheit 26 zusammen mit Start- bzw. Stoppsignalen vom Bildschirm 18, und zusätzlich bietet der Bildschirm 18 in nachfolgend zu beschreibender Weise die Möglichkeit, vor einem Beginn der Messungen über die erforderlichen Handhabungen bzw. die geforderten Reaktionen informiert zu werden.

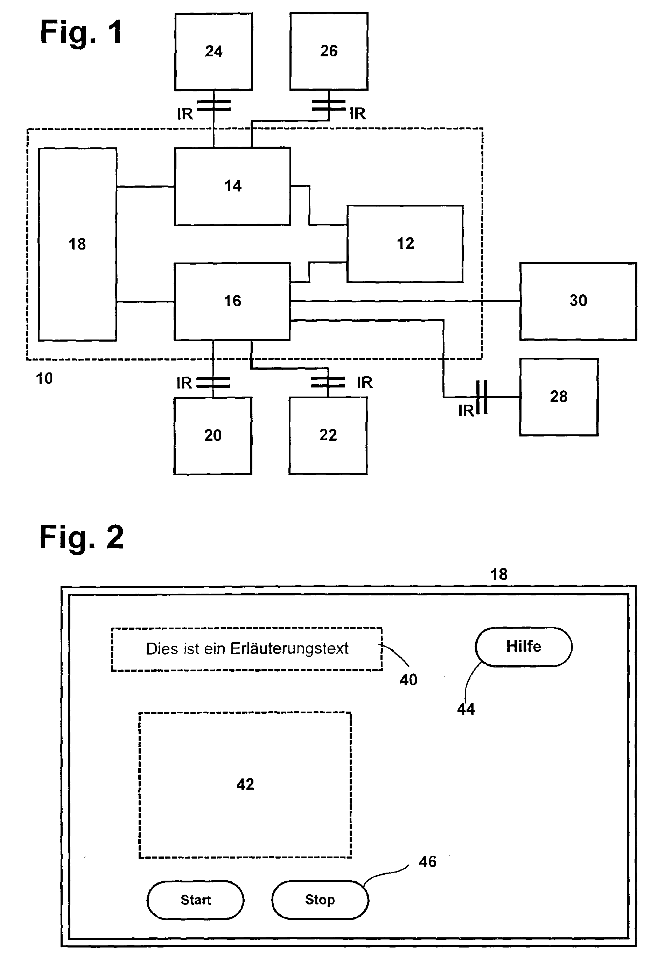

In Fig. 1 nicht gezeigt ist eine der zentralen Systemeinheit 12 zugeordnete Datenkommunikationseinheit, die in ansonsten bekannter Weise zum Herstellen einer Intra- oder Internetverbindung zu einer externen Servereinheit vorgesehen ist, und welche neben der Aktualisierung von Betriebssoftware der in Fig. 1 gezeigten Einheit insbesondere auch den Austausch von Messdaten zwischen der lokal betriebenen Einheit und der Servereinheit, eingeschlossen das Übertragen aktuell erfasster Datenparameter zum Server sowie das Empfangen von (an anderen Stellen ermittelten) Vergleichsdaten.

Unter Bezug auf die Fig. 2, die in schematisch vereinfachter Weise die Anzeigefläche des Bildschirms 18, so wie sie der Testperson anboten wird, zeigt, sollen verschiedene Möglichkeiten beschrieben werden, mit der Testperson visuell zu kommunizieren. So sind durch geeignete Ansteuerung von entsprechenden Bildschirmflächen sowohl eine Start- als auch eine Stopptaste 46 darstellbar, die, etwa durch geeignete Farbgebung, einen Farbwechsel od. dgl., den Beginn einer Reaktionsperiode signalisieren können, und welche dann die Testperson (als Reaktion auf den Reiz) zu berühren hat, um die Reaktionszeitmessung zu beenden. Selbstverständlich lassen sich die schematisch gezeigten Flächen 46 in beliebiger Weise ausgestalten, eingeschlossen das Vorsehen einer Mehrzahl von (z. B. je einer vorbestimmten Reihenfolge durchnumerierten) Flächen, welche dann ebenfalls in der numerierten Reihenfolge durch die Testperson zu berühren sind, um die Messung erfolgreich abzuschließen.

Zusätzlich bietet der Bildschirm 18 die Möglichkeit an, in flexibler Weise das System durch Hilfetexte zu ergänzen, bzw. es da selbsterklärend zu gestalten: So ist es beispielsweise möglich, dass durch Berühren einer Hilfefläche 44 die Testperson einen Erläuterungstext (schematisch gezeigt unter 40) anfordert, einen in einem Flächenbereich 42 darstellbaren Videoclip über die betreffende Messung anfordert, oder auf weitere Weise mit dem System kommuniziert (ohne dass, wie vorteilhaft realisierbar ist, eine zusätzliche Person zu Erklärungs- bzw. Einweisungszwecken zwingend anwesend sein muss).

So liegt ein bevorzugter Einsatz der in den Figuren beschriebenen Ausführungsform der Erfindung insbesondere auch darin, einer Testperson verschiedene Betriebsmodi anzubieten, die idealerweise nacheinander abgearbeitet werden sollten: Ein Demonstrationsmodus, wo etwa unter Zuhilfenahme der Erläuterungstexte und/oder Bildsequenzen der Person der Test, etwaige Hintergründe und konkret vorzunehmende Schritte erläutert werden. Daraufhin könnte die Testperson dann einen Trainingsmodus aktivieren, nämlich, ohne dass konkrete Werte für die Weiterverarbeitung erfasst werden, die erwarteten Reaktionen testen und sich so auf die von ihr erwarteten Schritte einstellen. In einem konkreten Messmodus würde dann eine vorbestimmte Anzahl von Messungen durchgeführt werden, wobei, je nach Konfiguration, ein Optimalwert einer Testperson für die Weiterverarbeitung erfasst wird, oder aber aus den Einzelversuchen der Testperson dann geeignete Mittelwerte gebildet werden.

Zur Ergänzung und weiteren Flexibilisierung des beschriebenen Ausführungsbeispiels ist vorgesehen, dem Probanden auch einen akustischen Reiz anzubieten, welcher insbesondere über eine mit der Ausgabeeinheit 16 (bevorzugt ebenfalls drahtlos mittels Infrarot) verbindbare Kopfhörereinheit 28 übermittelt werden könnte. Wird zudem gemäß einer bevorzugten Ausführungsform der Erfindung die einem Benutzer angebotene Unterstützung mittels Hilfetexten und/oder bewegten Bildern durch ein Audiosignal, z.B. einen gesprochenen Anleitungstext in einer entsprechend zugehörigen Audiodatei, implementiert, so ist es im Rahmen der vorliegenden Erfindung bevorzugt, im Gehäuse 10 einen (in den Figuren nicht gezeigten Lautsprecher vorzusehen, welcher dann insbesondere für eine entsprechende Sprachausgabe an einen Benutzer bzw. eine Testperson vorgesehen ist.

Für bevorzugt Benutzer-individuallisierte Ausdrucke, die, weiter bevorzugt, dem Probande auf visuelle Weise eine Einordnung seiner Leistungsdaten in eine zugehörige Bezugsgruppe ermöglichen, sorgt dann ein durch eine Druckereinheit 30 erzeugter Ausdruck, wobei auch hier die Anpassungs-und Variationsmöglichkeiten, je nach Konfiguration und Programmierung, nahezu unbegrenzt sind.

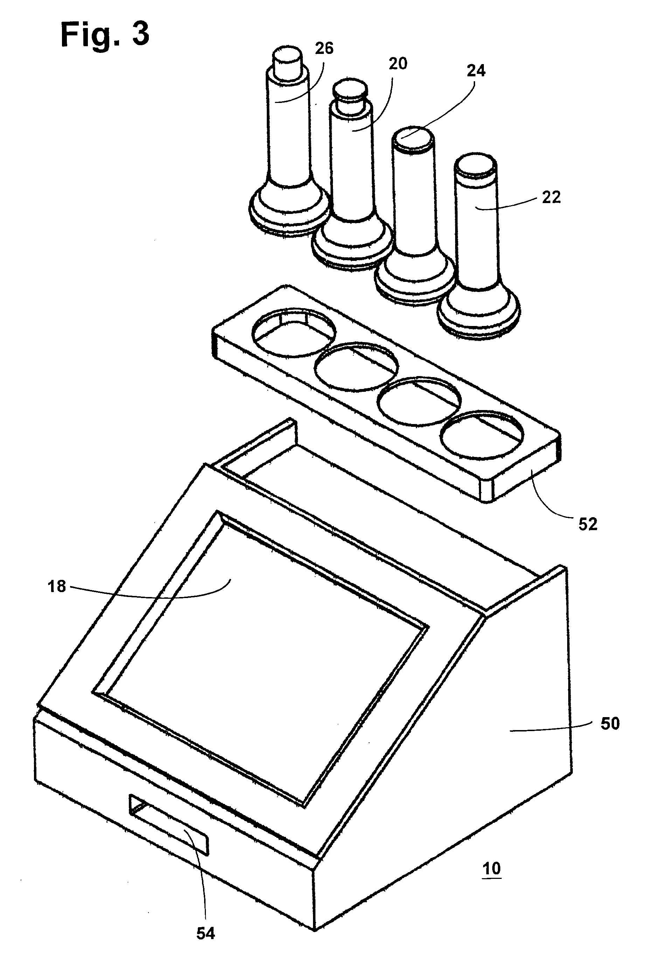

Unter Bezug auf die Fig. 3 soll beschrieben werden, wie die in Fig. 1 bzw. 2 schematisch beschriebene Ausführungsform mechanisch-konstruktiv realisiert wird, nämlich insbesondere Gestaltung und Aufbau des Pultgehäuses 10 sowie der externen Handeinheiten 20 bis 26.

Wie aus der Fig. 3 zu erkennen ist, bietet das Pultgehäuse 10 an seiner einem Benutzer zugewandten Schrägfläche einen Ausschnitt für den unterliegenden, berührungs- bzw. druckempfindlichen Bildschirm 18 an, so dass der Benutzer, mit Ausnahme der Bildschirmoberfläche, keine weiteren Bedienungselemente zu beachten bzw. handzuhaben hat; dies erleichtert nicht nur eine störungsfreie, einfache Bedienung und Ablaufsteuerung, auch wird Reinigung und Hygieneverhalten der Vorrichtung deutlich verbessert.

Wie aus der Explosionsansicht der Fig. 3 zudem erkennbar ist, werden die Handeinheiten 20 bis 26 in einem abnehmbaren, geeignete Ausschnitte anbietenden Aufsatz 52 für das Pultgehäuse 50 gehalten, und zwar so, dass in einem aufgesetzten Zustand (d. h. bei Nicht-Benutzung der Handeinheiten) diese übersichtlich, leicht zugreifbar und sicher gehalten sind.

Im praktischen Betrieb erfolgt dann, wie oben bereits dargestellt, eine Handhabung dieser Handeinheiten durch die Testperson, wobei die für einen jeweiligen Testzweck eingesetzten Einheiten dann drahtlos mit der im Gehäuse 10 vorgesehenen Systemeinheit 12 mittels einer IrDA-Schnittstelle kommunizieren, die in geeigneter Weise Infrarot-Signale durch einen in einer Stirnseite des Gehäuses 10 gebildeten Ausschnitt 54 empfängt.

Wie zudem aus der Fig. 3 deutlich wird, sind die glocken-bzw. stößelförmig geformten Handeinheiten 20 bis 26 mittels eines zylindrischen Längsabschnittes realisiert, welcher von der Bedienperson mit der Hand umgriffen wird, und einends ist dann eine glockenförmige Erweiterung vorgesehen, welche nicht nur dem besseren Stand auf dem Gehäuse 10 dient, sondern zusätzlich Platz zum Aufnehmen von interner Elektronik bzw. von Sensorelementen bietet. Der Gehäuseaufsatz 52 nimmt darüber hinaus auch (in der Fig. 3 nicht gezeigt) Lade- bzw. Versorgungselektronik für in den Handeinheiten 20 bis 26 aufgenommene Akkus auf, die im Drahtlosbetrieb die Stromversorgung sicherstellen. Gemäß einer weiteren bevorzugten Weiterbildung kann zudem der Aufsatz 52 neben dem Gehäuse plaziert werden.

Bevorzugt ist es zudem, neben einer durch den Sensorzweck vorgegebenen Ausgestaltung der Handeinheiten -- so ist, wie erwähnt, etwa die Handkrafteinheit 24 zum besseren Aufnehmen bzw. Messen einer durch Hand aufgebrachten Druckkraft geschlitzt -- die Handeinheiten mit optisch gut erkennbarem, unterscheidungskräftigen Markern zu versehen; im beschriebenen Ausführungsbeispiel werden auf den zylindrischen Abschnitt der Handeinheiten geeignet gebildete, weiter bevorzugt farbig eloxierte Aluminiumringe aufgesetzt.

Im Ergebnis ermöglicht es die vorbeschriebene Erfindung daher, die im praktischen Betrieb deutlich werdenden Nachteile existierender Vorrichtungen zur Vitalitätsdiagnostik zu überwinden und interessierten Personenkreisen ein System anzubieten, welches einfache Installation mit komfortabler Bedienung, erhöhter Akzeptanz bei Testpersonen und größerer Flexibilität beim Erstellen und Auswerten von vitalitätsrelevanten Versuchsmaßnahmen anbietet.

## Patentansprüche

1. Vorrichtung zur Vitalitätsdiagnostik an einer Testperson, mit
einer einer Datenverarbeitungsvorrichtung (12) zugeordneten Ausgabeeinheit (14, 18), die zum Ausgeben eines visuellen und/oder akustischen Signals für die Testperson ausgebildet ist, sowie
einer Betätigungs- und Sensoreinrichtung (16, 18), die zum Erfassen einer Eingabebetätigung der Testperson als Reaktion auf das visuelle oder akustische Signal eingerichtet ist,
wobei ein Ausgangssignal der Betätigungs- und Sensoreinrichtung durch die Datenverarbeitungsvorrichtung datenmäßig erfasst und zur Ermittlung vitalitätsrelevanter Daten weiter verarbeitet wird,
**dadurch gekennzeichnet, dass**
die Betätigungs- und Sensoreinrichtung sowie die Ausgabeeinheit einen gemeinsamen Berührungs- und/oder druckempfindlichen Bildschirm (18) aufweisen
und die Datenverarbeitungsvorrichtung mit dem Bildschirm in einem Gehäuse (10) integriert ist.

2. Vorrichtung nach Anspruch 1, **dadurch gekennzeichnet, dass** die Betätigungs- und Sensoreinrichtung mit einer zusätzlichen, manuell handhabbaren Detektoreinheit (24, 26) signal- und/oder datenmäßig verbunden ist, die zum Betätigen durch die Testperson sowie zum Erfassen von auf die Detektoreinheit wirkender Handkraft oder von durch Einblasen in die Detektoreinheit erzeugte Luftdruck ausgebildet ist.

3. Vorrichtung nach Anspruch 1 oder 2, **dadurch gekennzeichnet, dass** die Ausgabeeinheit mit einer zusätzlichen, manuell handhabbaren Vibrationseinheit (20) signal- und/oder datenmäßig verbunden ist, die zum Erzeugen eines bevorzugt in Frequenz oder Hub variabel steuerbaren, durch die Testperson fühlbaren Vibrationssignals ausgebildet ist.

4. Vorrichtung nach Anspruch 2 oder 3, **dadurch gekennzeichnet, dass** die Detektoreinheit und/oder die Vibrationseinheit mittels einer drahtlosen, bevorzugt auf Infrarotbasis wirkenden Verbindung (IR) mit der Betätigungs- und Sensoreinrichtung verbunden ist.

5. Vorrichtung nach einem der Ansprüche 1 bis 4, **dadurch gekennzeichnet, dass** die Datenverarbeitungsvorrichtung zum Steuern der Betätigungs- und Sensoreinrichtung sowie der Ausgabeeinheit so ausgebildet ist, dass die Ausgabeeinheit auf einem Teilbereich des druckempfindlichen Bildschirms (18) ein visuell sichtbares Signal (46) erzeugt und die Betätigungs- und Sensoreinrichtung zum Erfassen einer Berührung des Teilbereichs durch die Testperson ausgebildet ist.

6. Vorrichtung nach Anspruch 5, **dadurch gekennzeichnet, dass** die Datenverarbeitungsvorrichtung zum Erfassen und Auswerten einer Reihenfolge einer Berührung einer Mehrzahl von Teilbereichen als Reaktion auf das Anzeigen einer entsprechenden Mehrzahl von visuell sichtbaren Signalen auf dem Bildschirm ausgebildet ist.

7. Vorrichtung nach einem der Ansprüche 1 bis 6, **dadurch gekennzeichnet, dass** die Datenverarbeitungsvorrichtung zum Wiedergeben einer bevorzugt animierten, eine Diagnostikmaßnahme erläuternden Audio-, Text- und/oder Bilddarstellung (40, 42) zur Bedienerführung auf dem Bildschirm (18) ausgebildet ist, die als Reaktion auf eine Berührung eines vorbestimmten Teilbereichs (44) auf dem Bildschirm durch die Testperson aktivierbar ist.

8. Vorrichtung nach Anspruch 7, **dadurch gekennzeichnet, dass** die Bilddarstellung eine Videosequenz aufweist.

9. Vorrichtung nach einem der Ansprüche 1 bis 8, **dadurch gekennzeichnet, dass** die Datenverarbeitungsvorrichtung Mittel zur Kommunikation mit einer über ein bevorzugt öffentliches Datenübertragungsnetz anbindbaren Servereinheit aufweist.

10. Vorrichtung nach einem der Ansprüche 1 bis 9, **dadurch gekennzeichnet, dass** die Ausgabeeinheit eine extern vom Gehäuse vorgesehene oder in das Gehäuse integrierte Druckereinheit (30) aufweist, die zum Ausgeben einer graphischen Repräsentation von durch die Datenverarbeitungsvorrichtung erzeugten, vitalitätsrelevanten Daten der Testperson eingerichtet ist.
